**Europäisches Patentamt**

**European Patent Office**

**Office européen des brevets**

(19)

(11) Veröffentlichungsnummer : **0 140 336**
**B1**

(12) # EUROPÄISCHE PATENTSCHRIFT

(45) Veröffentlichungstag der Patentschrift :
07.06.89

(51) Int. Cl.⁴ : **C 12 M   1/40**

(21) Anmeldenummer : 84112823.4

(22) Anmeldetag : 24.10.84

(54) **Vorrichtung zur Herstellung von Biokatalysatorperlen.**

(30) Priorität : 03.11.83 DE 3339764

(43) Veröffentlichungstag der Anmeldung :
08.05.85 Patentblatt 85/19

(45) Bekanntmachung des Hinweises auf die Patenterteilung : 07.06.89 Patentblatt 89/23

(84) Benannte Vertragsstaaten :
AT BE CH DE FR GB IT LI LU NL SE

(56) Entgegenhaltungen :
DE-A- 2 835 875
DIE ANGEWANDTE MAKROMOLEKULARE CHEMIE,
Band 76/77, Nr. 1141, 1979, Seiten 329-350, Basel, CH;
J. KLEIN et al.: "Polymernetzwerke zum Einschluss
von Mikroorganismen"
AGRICULTURAL & BIOLOGICAL CHEMISTRY, Band
43, Nr. 5, Mai 1979, Seiten 1133-1138, Tokyo, JP; O.
MIYAWAKI et al.: "Microencapsulation of urease by
interfacial polymerization with liquid-air nozzle
method"

(73) Patentinhaber : **B. Braun-SSC AG**
**Gerliswilstrasse 74**
**CH-6020 Emmenbrücke (CH)**

(72) Erfinder : **Kahlert, Wolfgang**
**Ernstbergstrasse 4**
**D-3508 Melsungen (DE)**
Erfinder : **Klein, Joachim, Prof. Dr.**
**Hühnerkamp 21**
**D-3300 Braunschweig (DE)**
Erfinder : **Steinert, Hans-Jürgen**
**Maschtor 3**
**D-3152 Ilsede 4 (DE)**
Erfinder : **Vorlop, Claus**
**Hochstrasse 7**
**D-3300 Braunschweig (DE)**

(74) Vertreter : **von Kreisler, Alek, Dipl.-Chem. et al**
**Deichmannhaus am Hauptbahnhof**
**D-5000 Köln 1 (DE)**

## Beschreibung

Die Erfindung betrifft eine Vorrichtung zur Herstellung von Biokatalysatorperlen, mit einem keimdicht abgeschlossenen Immobilisierungsgefäß, das in seinem oberen Bereich mindestens einen Einfüllstutzen und in seinem unteren Bereich Auslaufrohre aufweist, die durch eine Druckkammer hindurchgehen, welche koaxial zu den Auslaufrohren angeordnete Ausströmöffnungen aufweist, und mit einem unter dem Immobilisierungsgefäß angeordneten Auffanggefäß zur Aufnahme einer Vernetzerlösung bzw. eines Umfällbades.

Immobilisierte Zellen sind wirksame Katalysatoren bei der enzymatischen Konversion organischer Bestandteile. Es ist bekannt, die Immobilisierung von Zellen durchzuführen, indem die Zellen zusammen mit einer Trägersubstanz in eine gelierfähige Polymerlösung eingegeben werden, die dann unter Tropfenbildung aus dem Immobilisierungsgefäß ausläuft und in ein Auffanggefäß fällt, das eine Vernetzerlösung enthält (J. Klein u. K.-D. Vorlop (1983) ACS Symposium Series 207, 377-392 ; American Chemical Society 1983, 17). Die Tropfen werden gebildet, indem koaxial zu den aus dem Immobilisierungsgefäß herausführenden Auslaufrohren sterile Druckluft zugeführt wird, die an den Auslaufrohren entlangstreicht und die Formung und Ablösung der Tropfen von dem Auslaufrohr erleichtert. Die Tropfen nehmen während des freien Falls und bei der Vernetzung Kugelform an, so daß in dem Auffanggefäß die Biokatalysatorperlen in Form von Kugeln vorhanden sind.

Von besonderer Bedeutung bei der Immobilisierung von Zellen ist die Sterilhaltung vor und während des Immobilisierungsprozesses. Bei Benutzung der bekannten Einrichtung müssen sämtliche Teile separat vorsterilisiert und anschließend in einer sterilen Atmosphäre verwendet werden.

Der Erfindung liegt die Aufgabe zugrunde, eine Vorrichtung der eingangs genannten Art zu schaffen, mit der die Herstellung von Biokatalysatorperlen unter sterilen Bedingungen erleichtert wird.

Zur Lösung dieser Aufgabe ist erfindungsgemäß vorgesehen, daß das Immobilisierungsgefäß einen die Gesamtheit der Auslaufrohre umgebenden unteren Anschlußstutzen aufweist und daß das Auffanggefäß keimdicht verschlossen ist und einen Deckel mit einer Öffnung zum abgedichteten Verbinden des Anschlußstutzens aufweist.

Hierbei bildet das Immobilisierungsgefäß zusammen mit dem Auffanggefäß eine Baugruppe, die insgesamt sterilisiert werden kann und bei der während des Gebrauchs insbesondere im Übergangsbereich zwischen Immobilisierungsgefäß und Auffanggefäß keine Kontaminationen durch die umgebende Luft erfolgen können. Beide Gefäße können dennoch separat hergestellt und auch separat gereinigt oder für andere Zwecke benutzt werden. Die Mikroorganismen oder Enzyme, die in den Biokatalysatorperlen eingeschlossen werden, kommen auch während des freien Falls in die Vernetzerlösung bzw. das Umfällbad nicht in Kontakt mit der Atmosphäre, so daß der gesamte Vorgang unter keimdichten Bedingungen stattfindet.

Das Auffanggefäß kann als Fermenter ausgebildet und mit einem Rührwerk und/oder einer Heizung ausgestattet sein. Hierdurch ist es möglich, das Auffanggefäß zugleich für die Weiterverarbeitung der Biokatalysatorperlen zu benutzen, ohne daß diese nach ihrer Herstellung noch in ein anderes Gefäß überführt werden müssen.

Gemäß einer bevorzugten Weiterbildung der Erfindung ist vorgesehen, daß die Auslaufrohre in einem demontierbaren ringförmigen Einsatz des Immobilisierungsgefäßes befestigt sind, der eine obere Lochplatte aufweist, in deren Löcher die oberen Enden der Auslaufrohre eingepaßt sind, und eine im Abstand von der oberen Lochplatte angeordnete untere Platte, in der die Ausströmöffnungen angeordnet sind. Der ringförmige Einsatz bildet hierbei ein demontierbares Teil, das zu Reinigungs- und Wartungszwecken von dem Immobilisierungsgefäß entfernt werden kann. Der Raum zwischen der oberen Lochplatte und der unteren Platte stellt die Druckkammer dar, in die ein steriles Gas eingegeben wird, das durch die unteren Ausströmöffnungen koaxial zu den Auslaufrohren entweicht und die Tropfenablösung an den unteren Enden der Auslaufrohre unterstützt sowie die Tropfengröße bestimmt.

Vorzugsweise ist der Einsatz von einem an eine Druckquelle angeschlossenen Ringraum umgeben und er weist in seiner Wand Öffnungen auf, die aus dem Ringraum in das Innere des Einsatzes führen. Hierdurch ist eine gleichmäßige Druckbeaufschlagung der Druckkammer gewährleistet, wobei die Verteilung des sterilen Druckgases in dem Ringraum außerhalb des Einsatzes erfolgt. Eine gleichmäßige Druckverteilung des strömenden Druckgases ist für die Erzielung gleichmäßiger Größen der Biokatalysatorperlen besonders wichtig.

Die Auslaufrohre ragen vorzugsweise bis unter den Deckel in das Innere des Auffanggefäßes hinein. Die Auslaufrohre stehen somit bis über das untere Ende des Immobilisierungsgefäßes hinaus vor, so daß die Tropfenablösung im Innern des Auffanggefäßes erfolgt.

Der apparative Aufbau zur Erzeugung von Biokatalysatoren kann dadurch vereinfacht werden, daß das Immobilisierungsgefäß ein von oben in seinen Innenraum hineinragendes Rührwerk aufweist bzw. mit einer Heizvorrichtung versehen ist. Hierdurch kann das Immobilisierungsgefäß gleichzeitig als Mischbehälter verwendet werden, in den die Trägersubstanz und die Enzyme bzw. Mikroorganismen separat eingegeben werden, wobei die Vermischung im Innern des Immobilisierungsgefäßes erfolgt. Hierdurch wird ein zusätzliches Mischgefäß zur Vorbereitung der Immobilisierung erspart. Um die Enzyme bzw. Mikroorganismen in das Immobilisierungsgefäß unter sterilen Bedingungen einführen zu können, weist das Immobilisierungsgefäß am oberen Ende einen Impfstutzen mit einer durchstechbaren Membran auf.

Im folgenden werden unter Bezugnahme auf die Zeichnungen Ausführungsbeispiele der Erfindung näher erläutert.

Es zeigen :

Fig. 1 einen Längsschnitt durch eine Kombination aus Immobilisierungsgefäß und Auffangbehälter,

Fig. 2 einen Längsschnitt durch eine andere Ausführungsform eines Immobilisierungsgefäßes und

Fign. 3 bis 5 schematische Darstellungen verschiedener Anwendungsarten der Vorrichtung.

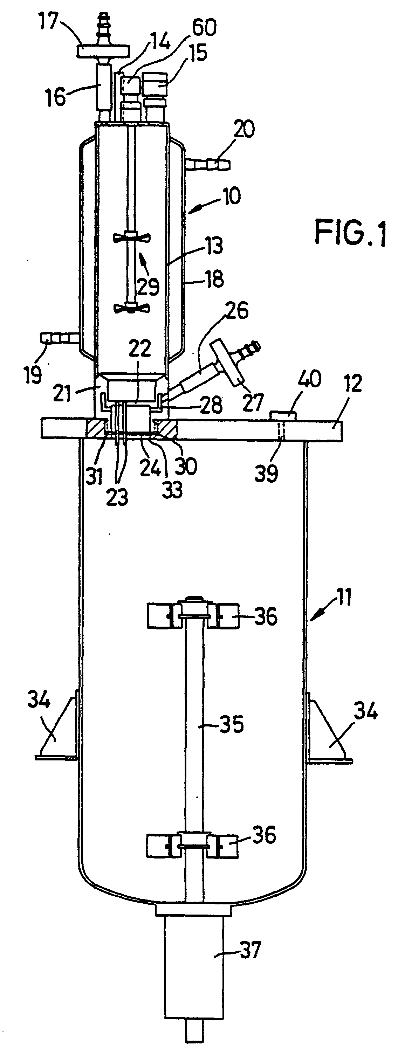

Gemäß Fig. 1 ist das Immobilisierungsgefäß 10 auf dem Deckel 12 des Auffanggefäßes 11 fest montiert. Das Immobilisierungsgefäß 10 besteht aus einem senkrechten rohrförmigen Behälter 13, in dessen oberer Stirnwand ein verschließbarer Einfüllstutzen 14, ein Impfstutzen 15, der eine mit einer Nadelkanüle durchstechbare Membran enthält, und ein Gaseinlaß 16 mit einem Filter 17 angeordnet sind. Der Einfüllstutzen 14 und der Gaseinlaß 16 sind Stutzen, die einander gleich ausgebildet sind und die auch hinsichtlich ihren Funktionen vertauscht oder zu anderen Zwecken benutzt werden können.

Die Umfangswand des Behälters 13 ist von einer Heizvorrichtung 18 in Form eines Heizmantels, der einen Einlaßstutzen 19 und einen Auslaßstutzen 20 für ein Heizmedium aufweist, umgeben. Das untere Ende des Behälter 13 ist durch einen Block 21 abgeschlossen. Der Block 21 weist eine horizontale Lochplatte 22 auf, die die untere Bodenwand des Behälters 13 bildet und in deren Löcher die oberen Enden zahlreicher vertikaler Auslaufrohre 23 eingepaßt sind. Die Auslaufrohre 23 ragen durch Löcher einer unteren Platte 24 hindurch bis in das Innere des Auffanggefäßes 11 hinein. Zwischen den Platten 22 und 24 ist eine Druckkammer 25 gebildet, in die über einen Gasanschluß 26 mit Filter 27 ein steriles Gas eingeleitet wird. Der Gasanschluß 26 führt in einen Ringraum 28 im Innern des Blockes 21 hinein. Von dem Ringraum 28 erstrecken sich Bohrungen in die Druckkammer 25.

Im Innern des Behälters 13 befindet sich ein Rührwerk 29, das entweder mit einem elektrischen Antrieb außerhalb des Behälters 13 verbunden ist oder an einem Drehknopf 60 von außen von Hand gedreht werden kann. Das Rührwerk besteht aus einer Rührwerkswelle und daran angebrachten Rührflügeln.

Das untere Ende des Blockes 21 des Immobilisierungsgefäßes 10 ist als Gewindestutzen 30 ausgebildet. Der Gewindestutzen 30 ist in eine Gewindebohrung des Deckels 12 eingeschraubt. Eine Dichtung 31 dichtet den Durchgang durch die Öffnung 33 des Deckels 12 ab.

Das Auffanggefäß 11, dessen Volumen erheblich größer ist als dasjenige des Immobilisierungsgefäßes, 10, besteht ebenfalls aus einem rohrförmigen Behälter, der an seiner Außenwand Halterungen 34 zur Befestigung an einer Abstützvorrichtung aufweist. Durch die Bodenwand des Auffanggefäßes 11 ragt eine Rührwerkswelle 35, die Rührflügel 36 trägt und von einem außerhalb des Auffanggefäßes 11 angeordneten Motor 37 antreibbar ist. Der Auffangbehälter 11 kann zusätzlich beheizbar sein. Er ist im vorliegenden Fall als Fermenter ausgebildet. Durch eine Entlüftungsöffnung 39 mit Filter 40 kann Überdruck aus dem Auffanggefäß 11 entweichen.

Die in Fig. 1 dargestellte Baugruppe aus Immobilisierungsgefäß 10 und Auffanggefäß 11 kann in dem dargestellten montierten Zustand insgesamt sterilisiert werden. Ihre Benutzung wird weiter unten noch erläutert werden.

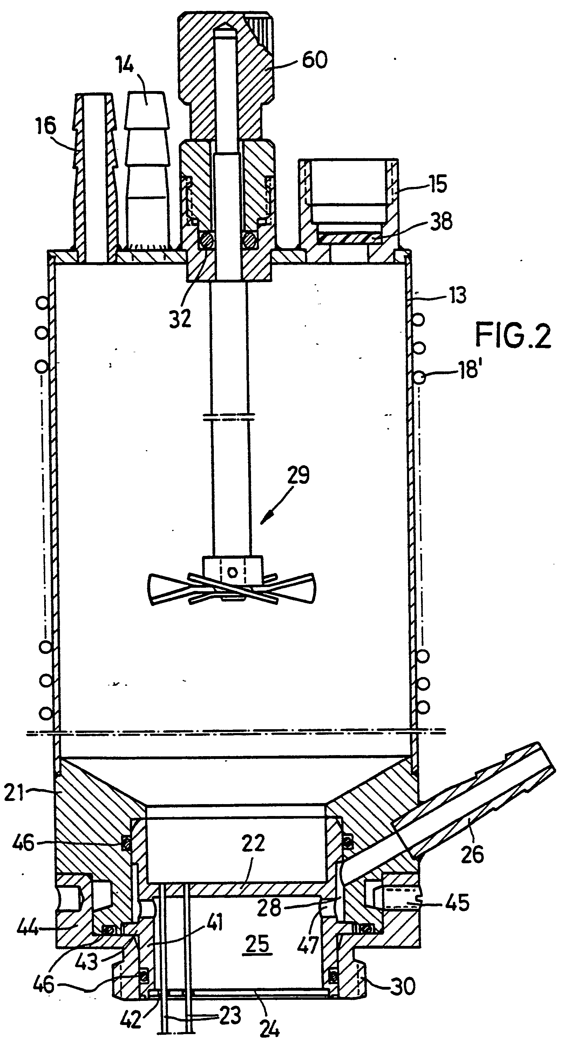

Das in Fig. 2 dargestellte Immobilisierungsgefäß unterscheidet sich nur geringfügig von demjenigen der Fig. 1 bzw. gibt dessen Konstruktionsdetails genauer an. In dem Impfstutzen 15 ist die aus Elastomermaterial bestehende durchstechbare Membran 38 erkennbar, während die Verschlußkappe abgenommen ist. Die Welle des Rührwerks 29 ist durch einen mit einer Dichtung 32 abgedichteten Durchgang aus der oberen Stirnwand des Behälters 13 nach außen geführt und dort mit einem manuell drehbaren Drehknopf 60 verbunden. Die Heizvorrichtung 18' besteht aus einer um den Behälter 13 schraubenförmig herumgewickelten Heizwendel.

Der Block 21, der an das untere Ende des Behälters 13 angeschweißt ist, enthält eine Axialbohrung, in die ein im wesentlichen rohrförmiger Einsatz 41 eingepaßt ist. Die Lochplatte 22 wird durch eine Zwischenwand des Einsatzes 41 gebildet und die untere Platte 24 bildet den unteren Abschluß des Einsatzes 41. In Fig. 2 sind auch die die Auslaufrohre umgebenden Ausströmöffnungen 42 erkennbar. Der Einsatz 41 weist einen umlaufenden Flansch 43 auf, der in eine Stufenausnehmung am unteren Ende des Blockes 21 eingepaßt ist. Gegen den Flansch 43 drückt von unten her eine Überwurfkappe 44, die mit radialen Madenschrauben 45 an dem Block 21 unter axialer Spannung festgelegt ist.

Der Ringraum 28, der einen Teil der Länge des Einsatzes 41 umgibt, besteht aus einer ringförmigen Ausnehmung am Umfang des Einsatzes 41. Zur Abdichtung des Ringraumes 28 dienen Ringdichtungen 46. Aus dem Ringraum 28 führen radiale Bohrungen 47 in die Druckkammer 25, durch die die Auslaufrohre 23 achsparallel hindurchgehen.

Zunächst wird im folgenden die Technik der Immobilisierung allgemein beschrieben :

Eine Trägersubstanz, beispielsweise eine viskose Polyelektrolytlösung (Alginat, Pektinat, Carboxymethylcellulose, Carrageenan, Furcellaran, Cellulosesulfat, Chitosan usw.), eine gelierfähige Polymerlösung (Curdlan, Agar, Agarose, Gelatine usw.) oder eine nichtwäßrige Polymerlösung (Celluloseacetat, Polystyrol, Eudragit usw.) wird mit den Enzymen, Zellorganellen oder ganzen Zellen gemischt. Diese Mischung wird dann in das temperierbare Immobilisierungsgefäß 10 gegeben bzw. gepumpt, und mit Druck beaufschlagt. Der Druck wird durch ein Manometer vorgewählt, das Gas strömt dabei durch das Sterilfilter 17. Die Mischung wird durch die dünnen Auslaufrohre 23 in die Vernetzerlösung bzw. das Umfällbad getropft, wobei sich momentan Biokatalysatorperlen ausbilden. Der seitliche untere Druckanschluß 26 ist über das Sterilfilter 27 und ein Manometer an eine Druckleitung angeschlossen. Dieser Gasstrom strömt an den Stahlröhrchen vorbei und reißt dabei die Tropfen, die sich an den Kanten der Auslaufrohre 23 gebildet haben, frühzeitig ab. Durch die Wahl des Druckes in der Druckkammer 25 kann die Tropfengröße exakt eingestellt werden. Es werden Perlen mit einer engen Radienverteilung erhalten.

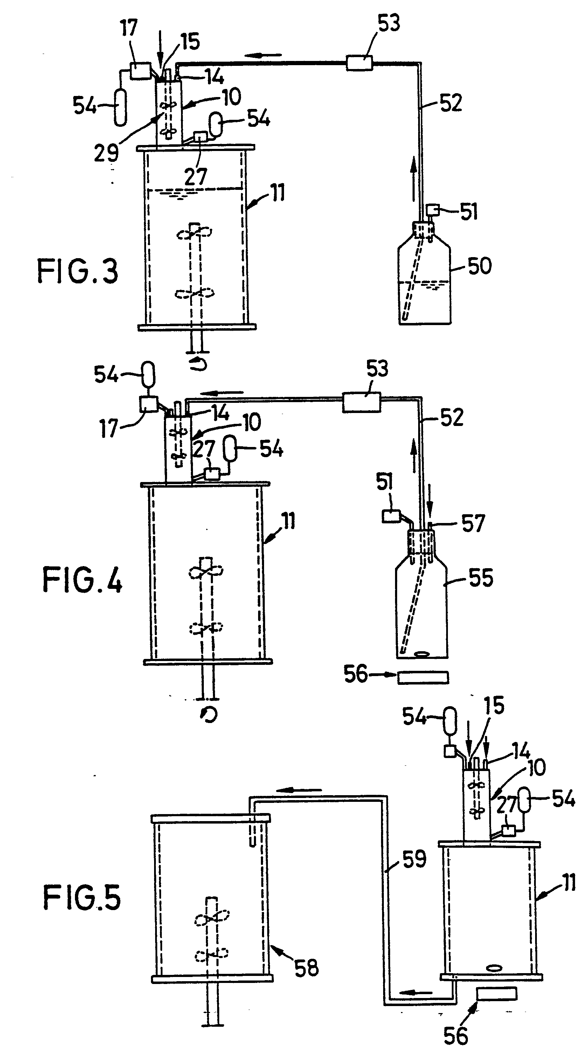

Die Fign. 3 bis 5 zeigen verschiedene Arbeitsabläufe, die unter Verwendung der Vorrichtung durchgeführt werden können.

Bei dem Arbeitsablauf der Fig. 3 werden zunächst das Immobilisierungsgefäß 10, das Auffanggefäß 11 und die Sterilfilter 17 und 27 gemeinsam sterilisiert. Die Trägersubstanz ist in einer sterilen Flasche 50 enthalten, die mit einem Sterilfilter 51 zur Belüftung ausgestattet ist. Aus der Flasche 50 führt ein Schlauch 52 über eine Pumpe 53, z. B. eine Schlauchpumpe, zu dem Einfüllstutzen 14 des Immobilisierungsgefäßes 10. Durch die Pumpe wird die Trägersubstanz in das Immobilisierungsgefäß gepumpt und durch den Impfstutzen 15 werden die Mikroorganismen bzw. Enzyme zugegeben. Beide Substanzen werden mit Hilfe des Rührwerks 29 vermischt. Das Auffanggefäß 11 wird somit zugleich als Fermentationsgefäß benutzt. Die an die Sterilfilter 17 und 27 angeschlossenen Gasdruckquellen sind in den Fign. 3 bis 5 mit 54 bezeichnet.

Der in Fig. 4 dargestellte Arbeitsablauf unterscheidet sich von demjenigen der Fig. 3 dadurch, daß die Trägersubstanz und die Enzyme, Zellorganellen oder ganzen Zellen in einem externen Gefäß 55, das einen Magnetrührer 56 aufweist, gemischt und danach durch die Schlauchleitung 52 und die Pumpe 53 durch den Einfüllstutzen 14 in das Immobilisierungsgefäß 10 gepumpt werden. Das Gefäß 55 weist einen Einlaß 57 zum Zuführen der Enzyme bzw. Mikroorganismen auf. Der Inhalt des Gefäßes 55 kann entweder batchweise oder kontinuierlich in das Immobilisierungsgefäß 10 gepumpt werden. Somit kann ein größeres Volumen als das des Immobilisierungsgefäßes verarbeitet werden. Bei der kontinuierlichen Perlenherstellung kann ggf. auf die separate Druckbeaufschlagung 17 verzichtet werden.

Auch bei dem Arbeitsablauf der Fig. 4 werden Immobilisierungsgefäß 10 und Auffanggefäß 11, das als Fermenter benutzt wird, zusammen mit den Sterilfiltern 17 und 27 gemeinsam sterilisiert. Die Sterilisierung des die Trägersubstanz enthaltenden Gefäßes 55 und des Überführungssystems 52, 53 erfolgt getrennt hiervon.

Bei dem Arbeitsablauf nach Fig. 5 ist ein separates Fermentationsgefäß 58 vorgesehen, in das die immobilisierten Mikroorganismen oder Enzyme aus dem Auffanggefäß 11 über eine Schlauchleitung 59 überführt werden. Zu diesem Zweck weist das Auffanggefäß 11 an seiner Bodenwand einen Anschluß für die Schlauchleitung 59 auf. In den Einfüllstutzen 14 des Immobilisierungsgefäßes 10 wird die Trägersubstanz eingefüllt und Enzyme, Mikroorganismen o. dgl. werden durch den Impfstutzen 15 hindurch eingeimpft. Das Auffanggefäß 11 enthält die Vernetzerlösung bzw. das Umfällbad. Nachdem sich im Auffanggefäß 11 die Biokatalysatorperlen gebildet haben, werden diese vom Boden des Auffanggefäßes abgesaugt und durch die Schlauchleitung 59 in das Fermentationsgefäß überführt.

Im folgenden werden zwei Immobilisierungsbeispiele beschrieben, die nach dem Arbeitsablauf der Fig. 4 erhalten wurden :

1. In Alginat

Zu 400 ml einer sterilen 3.5 %igen Na-Alginat-Lösung (Protonal LF 20/60) wurden 100 ml einer Acetobacter sp.-Zellsuspension mit einem Biofeuchtmasseanteil von 80 g gegeben und gut suspendiert. Diese Suspension wurde anschließend in die beschriebene Immobilisierungsapparatur überführt. Durch Druckbeaufschlagung (1, 4 bar) mit steriler Luft wurde die Suspension durch die Stahlröhrchen in eine im Auffanggefäß befindliche 2 %ige-CaCl$_2$-Vernetzerlösung getropft. Hierbei kam es momentan zur Bildung von Ca-Alginat-Biokatalysatorperlen. Der sterile Luftstrom zum Abblasen der Perlen wurde dabei so gewählt, daß Perlen mit einem Durchmesser von 1.2 mm erhalten wurden. Nach einer Vernetzungszeit von 30 min wurden die Biokatalysatorperlen zur Herstellung von Gluconsäure aus Sauerstoff und Glucose eingesetzt. Die erhaltenen Biokatalysatoren hatten eine Aktivität von 3.88 U/g KFM bei einer Restaktivität von 48 %.

2. In Chitosan

Zu 400 ml einer sterilen 1.1 %igen Chitosan-Acetat-Lösung (Chitosan-hv) wurden 100 ml einer E. coli-Zellsuspension mit einem Biofeuchtmasseanteil von 50 g gegeben und gut suspendiert. Diese Suspension wurde anschließend in die beschriebene Immobilisierungsapparatur überführt. Durch Druckbeaufschlagung (1,3 bar) mit steriler Luft wurde die Suspension durch die Stahlröhrchen in die 2 %ige Na-Tripolyphosphat-Vernetzerlösung (pH 8) getropft. Der sterile Luftstrom zum Abblasen der Perlen wurde dabei so gewählt, daß Perlen mit einem Enddurchmesser von 0.6 mm erhalten wurden. Nach der vollständigen Durchvernetzung, Aushärtung und Schrumpfung der Chitosan-Biokatalysatorperlen konnten sie direkt zur Spaltung von Penicillin G zu 6-Aminopenicillansäure und Phenylessigsäure eingesetzt werden. Die erhaltenen Biokatalysatorperlen hatten ein Aktivität von 60 U/g KFM bei einer Restaktivität von 63 %.

**Patentansprüche**

1. Vorrichtung zur Herstellung von Biokatalysatorperlen, mit einem keimdicht abgeschlossenen Immobilisierungsgefäß (10), das in seinem oberen Bereich mindestens einen Einfüllstutzen (14) und in seinem unteren Bereich Auslaufrohre (23) aufweist, die durch eine Druckkammer (25) hindurchgehen, welche koaxial zu den Auslaufrohren (23) angeordnete Ausströmöffnungen (42) aufweist, und mit einem unter dem Immobilisierungsgefäß (10) angeordneten Auffanggefäß (11) zur Aufnahme einer Vernetzerlösung bzw. eines Umfällbades, dadurch gekennzeichnet, daß das Immobilisierungsgefäß (10) einen die Gesamtheit der Auslaufrohre (23) umgebenden unteren Anschlußstutzen (30) aufweist und daß das Auffanggefäß (11) keimdicht verschlossen ist und einen Deckel (12) mit einer Öffnung (33) zum abgedichteten Verbinden des Anschlußstutzens (30) aufweist.

2. Vorrichtung nach Anspruch 1, dadurch gekennzeichnet, daß das Auffanggefäß (11) ein Fermenter mit einem Rührwerk (35, 36) und/oder einer Heizung ist.

3. Vorrichtung nach Anspruch 1 oder 2, dadurch gekennzeichnet, daß die Auslaufrohre (23) in einem demontierbaren ringförmigen Einsatz (41) des Immobilisierungsgefäßes (10) befestigt sind, der eine obere Lochplatte (22) ausweist, in deren Löcher die oberen Enden der Auslaufrohre (23) eingepaßt sind, und eine im Abstand von der oberen Lochplatte (22) angeordnete untere Platte (24), in der die Ausströmöffnungen (42) angeordnet sind.

4. Vorrichtung nach Anspruch 3, dadurch gekennzeichnet, daß der Einsatz (41) von einem an eine Gasdruckquelle (54) angeschlossenen Ringraum (28) umgeben ist und in seiner Wand Öffnungen (47) aufweist, die aus dem Ringraum in das Innere des Einsatzes (41) führen.

5. Vorrichtung nach einem der Ansprüche 1 bis 4, dadurch gekennzeichnet, daß die Auslaufrohre (23) bis unter den Deckel (12) in das Innere des Auffanggefäßes (11) hineinragen.

6. Vorrichtung nach einem der Ansprüche 1 bis 5, dadurch gekennzeichnet, daß das Immobilisierungsgefäß (10) ein von oben in seinen Innenraum hineinragendes Rührwerk (29) aufweist.

7. Vorrichtung nach einem der Ansprüche 1 bis 6, dadurch gekennzeichnet, daß das Immobilisierungsgefäß (10) eine Heizvorrichtung (18, 18') aufweist.

8. Vorrichtung nach einem der Ansprüche 1 bis 7, dadurch gekennzeichnet, daß das Immobilisierungsgefäß (10) am oberen Ende einen Impfstutzen (15) mit einer durchstechbaren Membran (38) aufweist.

## Claims

1. An apparatus for producing biocatalyst pearls, comprising a germ-tightly closed immobilisation container (10), provided in its upper part with at least one feed-in socket (14) and in its lower part outlet tubes (23) extending through a pressure chamber (25) provided with flow-out openings (42) coaxially arranged to said outlet tubes (23), and a collecting basin (11) arranged below the immobilisation container (10), for accommodating a cross-linking solution or a precipitation bath, characterised in that the immobilisation container (10) is provided with a lower connection member (30) enclosing all of said outlet tubes (23) and that the collecting basin (11) is closed in germ-tight manner and is provided with a lid (12) having an opening (33) for sealingly connecting with the connection member (30).

2. The apparatus according to Claim 1, characterised in that the collecting basin (11) is a fermenter provided with an agitator (35, 36) and/or a heating.

3. The apparatus according to Claim 1 or 2, characterised in that the outlet tubes (23) are fastened in a demountable annular insert (41) of the immobilisation container (10), said annular insert comprising an upper perforated plate (22) into the perforations of which the upper ends of the outlet tubes (23) are fit, and a lower plate (24) spaced from the perforated plate (22), and comprising said flow-out openings (42) are provided.

4. The apparatus according to Claim 3, characterised in that the insert (41) is surrounded by an annular space (28) connected to a gas pressure source (54), and the wall of said insert comprising openings (47) that lead from said annular space into the inside of the insert (41).

5. The apparatus according to any one of Claims 1 to 4, characterised in that the outlet tubes (23) protrude into the collecting basin (11) to below the lid (12).

6. The apparatus according to any one of Claims 1 to 5, characterised in that the immobilisation container (10) is provided with an agitator (29) extending into the interior of the container from above.

7. The apparatus according to any one of Claims 1 to 6, characterised in that the immobilisation container (10) is provided with a heating means (18, 18').

8. The apparatus according to any one of Claims 1 to 7, characterised in that the upper end of the immobilisation container (10) is provided with a vaccination socket (15) having a pierceable diaphragm (38).

## Revendications

1. Appareil pour la fabrication de biocatalyseurs sous forme de perles comprenant une cuve d'immobilisation (10) close, de façon étanche aux germes, qui comporte dans sa zone supérieure au moins une tubulure de chargement (14) et dans sa zone inférieure des tuyaux d'évacuation (23) qui traversent une chambre sous pression (25) comportant des orifices de dégagement (42) coaxiaux aux tuyaux d'évacuation (23), et une cuve de réception (11) placée au-dessous de la cuve d'immobilisation (10) pour recevoir une solution de réticulant, respectivement un bain de précipitation, caractérisé en ce que la cuve d'immobilisation (10) comporte une tubulure de jonction inférieure (30) entourant l'ensemble des tuyaux d'évacuation (23) et que la cuve de réception (11) est fermée de façon étanche aux germes et comporte un couvercle (12) avec un orifice pour la liaison étanche de la tubulure de jonction (30).

2. Appareil selon la revendication 1, caractérisé en ce que la cuve de réception (11) est un fermenteur muni d'un agitateur mécanique (35, 36) et/ou d'un chauffage.

3. Appareil selon la revendication 1 ou 2, caractérisée en ce que les tuyaux d'évacuation (23) sont fixés dans une pièce d'insertion (41) annulaire démontable de la cuve d'immobilisation (10) qui comporte une plaque perforée supérieure (22) dans les trous de laquelle s'insèrent les extrémités supérieures des tuyaux d'évacuation (23) et une plaque inférieure (24) espacée de la plaque perforée supérieure (22) dans laquelle sont disposés les orifices de dégagement (42).

4. Appareil selon la revendication 3, caractérisé en ce que la pièce d'insertion (41) est entourée d'un espace annulaire (28) relié à une source de gaz comprimé (54) et présente dans sa paroi des ouvertures (47) conduisant, à partir de l'espace annulaire, à l'intérieur de la pièce d'insertion (41).

5. Appareil selon l'une des revendications 1 à 4, caractérisé en ce que les tuyaux d'évacuation (23) s'étendent à l'intérieur de la cuve de réception (11) jusqu'en dessous du couvercle (12).

6. Appareil selon l'une des revendications 1 à 5, caractérisé en ce que la cuve d'immobilisation (10) comporte un agitateur mécanique (29) pénétrant, par le haut, dans son espace intérieur.

7. Appareil selon l'une des revendications 1 à 6, caractérisé en ce que la cuve d'immobilisation (10) est équipée d'un dispositif de chauffage (18, 18').

8. Appareil selon l'une des revendications 1 à 7, caractérisé en ce que la cuve d'immobilisation (10) comporte à sa partie supérieure une tubulure d'ensemencement (15) pourvue d'une membrane perforable (38).

FIG.1

EP 0 140 336 B1

FIG.2

FIG.3

FIG.4

FIG.5